# EUROPÄISCHE PATENTSCHRIFT

(11) **EP 0 908 169 B1**
(45) Veröffentlichungstag und Bekanntmachung des Hinweises auf die Patenterteilung: **14.12.2005**
(21) Anmeldenummer: 98117147.3
(22) Anmeldetag: 10.09.1998
(51) Int. Cl.: A61K 6/00, A61L 26/00, A61L 15/34

(54) **Mischung zur Verwendung als Wundverband**
Composition for use as a wound dressing
Composition pour pansements liquides

(30) Priorität: 09.10.1997 DE 19744621
(43) Veröffentlichungstag der Anmeldung: 14.04.1999
(73) Patentinhaber: Ernst Mühlbauer GmbH & Co.KG, 25870 Norderfriedrichskoog (DE)
(72) Erfinder: Mühlbauer, Wolfgang, Dr., 22609 Hamburg (DE); Lück, Rainer SC, Dr., 25436 Tornesch (DE)
(74) Vertreter: Glawe, Delfs, Moll

(56) Entgegenhaltungen:
- WO-A-96/37227
- DE-A- 4 212 862
- DE-C- 959 051
- DE-C- 4 240 713
- FR-A- 2 705 567
- GB-A- 807 768

## Beschreibung

Gegenstand der Erfindung ist eine Mischung zur Verwendung als medizinischer Wundverband.

Aus offenkundiger Vorbenutzung ist die Verwendung calciumhydroxidhaltiger Präparate in der Zahnheilkunde bekannt. Sie dienen hauptsächlich als Wundverband, bspw. zur Versorgung von Knochentraumen oder zur Herstellung einer Wurzelkanalfüllung.

Aus DE 42 40 713 C1 ist es bekannt, daß eine Mischung von Calciumhydroxid und Rinderklauenöl die Kollagenneubildung in vivo unterstützen kann. Diese patentgemäße Paste erweist sich als nicht lagerstabil.

Bei der Knochenbildung erfolgt zuerst die Synthese von organischem Gewebe (Kollagen-Synthese) und daran anschließend die durch die sogenannte Matrixvesikel vermittelte Einlagerung von mineralischer Substanz in die organische Matrix. Das Bindegewebsprotein Kollagen ist die Hauptkomponente der organischen Substanz des Knochens. Das Protein besteht aus drei helikal gewundenen Polypeptidketten, deren Aminosäuresequenzen variieren können, was zu einer Vielfalt einzelner Kollagentypen führt. Allen Kollagentypen gemeinsam ist eine außerordentlich hohe mechanische Festigkeit der Kollagenfaser. Diese Festigkeit wird durch eine Vielzahl von intra- und intermolekularen Bindungen der Peptidketten aufgebaut, welche auf diese Weise das dichte Kollagenfaser-Netzwerk des Bindegewebes bilden. Diese Bindungen entstehen durch die Oxidation des Lysins und der anschließenden Reaktion des gebildeten Aldehyds mit freien Aminogruppen benachbarter Ketten. Daneben treten noch Wasserstoffbrückenbindungen und Esterbindungen mit Zukkerresten auf. Das Knochengewebe wird durch die Einlagerung von mineralischen Substanzen (Hydroxylapatit, Calciumphosphat) in dieses Netzwerk gebildet.

Mischungen aus Calciumhydroxid und Rinderklauenöl werden auch als Wurzelkanalfüllungen verwendet (DE 29 32 738 C2) und in Mischung mit Carboxylatzementen als Bestandteil temporärer Befestigungsmittel für provisorische Zahnstumpfabdeckungen (DE 34 13 864 C1). In beiden Fällen nutzt man die pulpitisprophylaktische Wirkung von Calciumhydroxid. Das Rinderklauenöl dient zum einen als Anteigmittel und verzögert zum anderen die Freisetzung des Calciumions und den Anstieg des pH-Wertes.

Die DE 40 40 713 C1 offenbart, daß Calciumhydroxid und Rinderklauenöl in Mischung nicht lagerstabil sind und unmittelbar vor der Anwendung angemischt werden müssen. Bei längerer Lagerung der Mischung verseift das Rinderklauenöl.

Der Erfindung liegt die Aufgabe zugrunde, eine Mischung zur Verwendung als Wundverband zu schaffen, die im applikationsfertigen Zustand lagerstabil ist. Erfindungsgemäß wird eine Mischung zur Verwendung als Wundverband geschaffen, die folgende Bestandteile enthält:
a) Paraffine nach DAB und/oder synthetische Wachse ausgewählt aus der Gruppe bestehend aus Montanwachsen, Wachsen aus Erdöl, Fischer-Tropsch-Wachsen, Polyolefinwachsen, Vaseline, Wachsalkoholen, und Oxidaten der vorgenannten Stoffe;
b) wenigstens ein Metallhydroxid.

Die erfindungsgemäße Mischung enthält kein Rinderklauenöl (Oleum pedum tauri).

Hinsichtlich der Definition der erfindungsgemäß verwendeten Wachse wird verwiesen auf Ullmanns Enzyklopädie der technischen Chemie, 4. Auflage, Verlag Chemie, Weinheim, Band 24, S. 16 - 45. Unter Oxidaten dieser synthetischen Wachse sind solche Stoffe zu verstehen, bei denen Sauerstoffunktionen (bspw. Carboxyl-, Ester-, Carbonyl- oder Hydroxylgruppen) nachträglich in die Wachsmoleküle eingeführt worden sind. Dies kann bspw. durch Umsetzen der Schmelze mit Luftsauerstoff geschehen. Im einzelnen wird hierzu ebenfalls auf den genannten Abschnitt des Ullmann verwiesen. Angemerkt sei noch, daß Vaseline ebenfalls als ein synthetisches Wachs anzusehen ist (siehe Ullmann a.a.O.).

Überraschenderweise ist eine erfindungsgemäße Mischung lagerstabil. Sie bewirkt jedoch in gleicher Weise wie eine Mischung aus Calciumhydroxid und Rinderklauenöl eine verzögerte Alkalitätsfreisetzung, die die Kollagenneubildung in vivo anregen kann und/oder eine pulpaprophylaktische Wirkung entfalten kann.

Der Begriff "Wundverband" ist im Rahmen der Erfindung weit zu verstehen und umfaßt insbesondere Anwendungen in der Kieferchirurgie, Implantologie, Traumatologie oder dergleichen, bei denen das erfindungsgemäße Gemisch auf oder in Knochentraumen wie bspw. Frakturflächen, Bohrungen, Kavitäten oder dergleichen appliziert wird. Der Begriff umfaßt bspw. die Wurzelfüllung sowie die Verwendung im Rahmen der Befestigung temporärer Zahnstumpfabdeckungen.

Bevorzugte synthetische Wachse im Rahmen der Erfindung sind Vaseline und bei Raumtemperatur flüssige Paraffine nach DAB (Deutsches Arzneimittelbuch), bspw. Paraffinum liquidum und Paraffinum perliquidum.

Die erfindungsgemäße Mischung kann zusätzlich pflanzliche Öle, Fette oder Wachse (Ester langkettiger Carbonsäuren mit langkettigen Alkoholen) enthalten. Diese zusätzlichen Stoffe können insbesondere bei der Einstellung der Konsistenz der üblicherweise pastenartigen Mischung hilfreich sein. Geeignete Pflanzenwachse sind bspw. Carnaubawachse, Candelillawachse, Ouricuriwachse, Zuckerrohrwachse, Retamowachse und auch Jojobaöl.

Unter Fetten und Ölen sind Stoffe zu verstehen, die Triglyceride, Diglyceride bzw. Monoglyceride als wesentliche Bestandteile enthalten. Hinsichtlich geeigneter pflanzlicher Fette und Öle wird verwiesen auf Ullmanns Enzyklopädie der technischen Chemie, 4. Auflage, Band 11, Seite 500 - 515. Gut geeignet sind bspw. Sonnenblumenöl und Rizinusöl.

Soweit die pflanzlichen Öle, Fette oder Wachse in Gegenwart eines Metallhydroxids eine gewisse Verseifungsneigung zeigen, die einer ausreichenden Lagerstabilität an und für sich abträglich wäre, bewirken überraschenderweise die in der erfindungsgemäßen Mischung vorhandenen synthetischen Wachse bzw. deren Oxidate eine Stabilisierung der Gesamtmischung, die zu einer ausreichenden Lagerstabilität führt.

Bevorzugt enthält die Mischung Jojobaöl, Rizinusöl, Maiskeimöl, Carnaubawachs und/oder Sonnenblumenöl. Als Metallhydroxide sind Alkali- und/oder Erdalkalihydroxide bevorzugt, besonders bevorzugt sind Magnesium- und/oder Calciumhydroxid. Der Anteil des Metallhydroxids oder der Metallhydroxide an der Mischung beträgt vorzugweise 10 - 75 Gew.-%.

Soweit die erfindungsgemäße Mischung pflanzliche Öle, Fette und/oder Wachse enthält, beträgt deren Anteil vorzugsweise 10 - 60 Gew.-%. Der Anteil synthetischer Wachse und/oder deren Oxidate liegt bevorzugt zwischen 10 und 50 Gew.-%.

Die erfindungsgemäße Mischung kann zusätzliche Füllstoffe aufweisen. Geeignet sind bspw. im Dentalbereich übliche Gläser oder Glaskeramiken, insbesondere Barium- oder Strontiumgläser oder Ionomergläser. Geeignet ist ferner ein glaskeramisches Fasermaterial, das unter dem Namen PRIMM (Polymeric Rigid Inorganic Matrix Material) bekannt ist (Leinfelder, JA-DA, **128** (1997) 573ff.).

Die genannte Aufzählung der Inhaltsstoffe der Mischung ist nicht notwendigerweise abschließend. Bspw. können zusätzlich Oxidationshemmer wie 2,6-Di-tert.-butyl-4-methylphenol zugesetzt werden. Andere biologisch verträgliche Zusatz- und Hilfsstoffe können ebenfalls hinzugefügt werden. Bspw. kann im Bedarfsfall Röntgenopazität durch Zusatz von Stoffen wie Bariumsulfat erreicht werden.

Durch Variation der den Metallhydroxiden zugesetzten Wachse, Fette und Öle sowie deren Mengenverhältnisse können Mischungen (üblicherweise in Pastenform) gewünschter Konsistenz und Handhabbarkeit bereitgestellt werden. Durch Wahl entsprechender Wachse bzw. Fette und/oder Öle als Anteigmittel können bspw. Pasten hergestellt werden, die als Wurzelkanalfüllung bzw. Knochenwundverband geeignet sind. Diese Pasten können in vivo bei Knochentraumata zur Förderung der Kollagenneubildung beitragen.

Die Erfindung wird nachfolgend anhand von Ausführungsbeispielen erläutert.

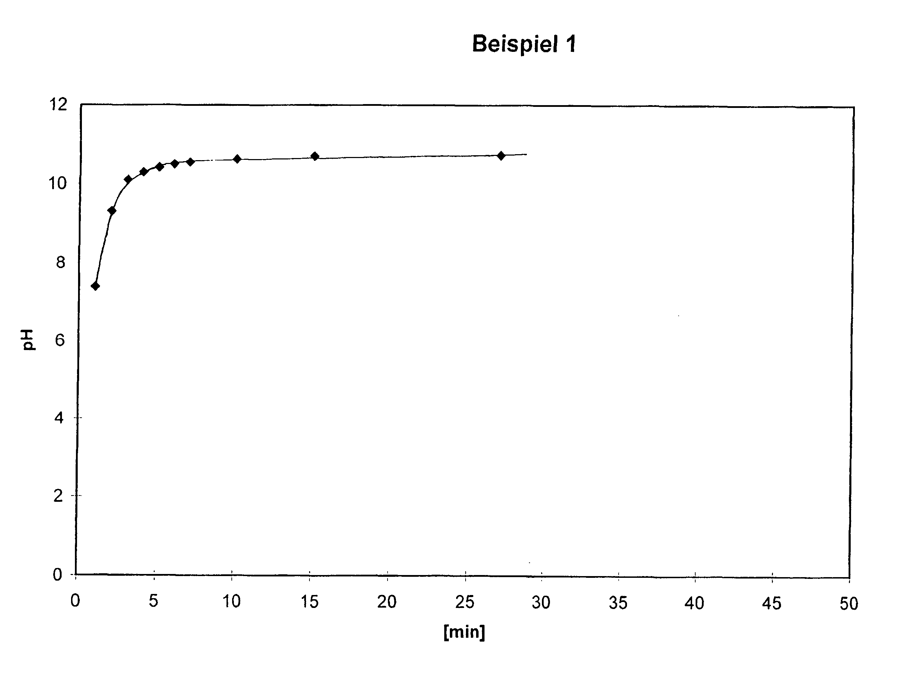

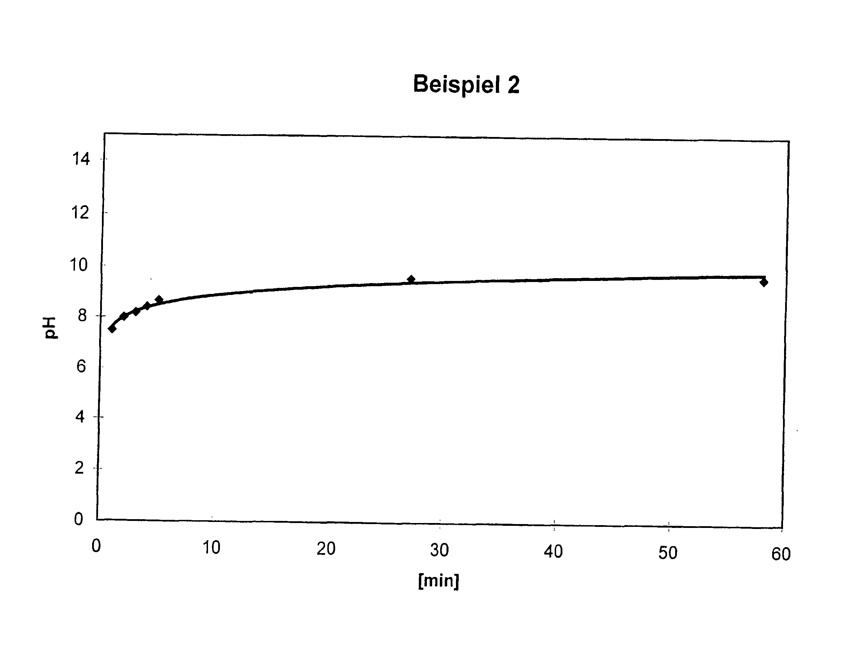

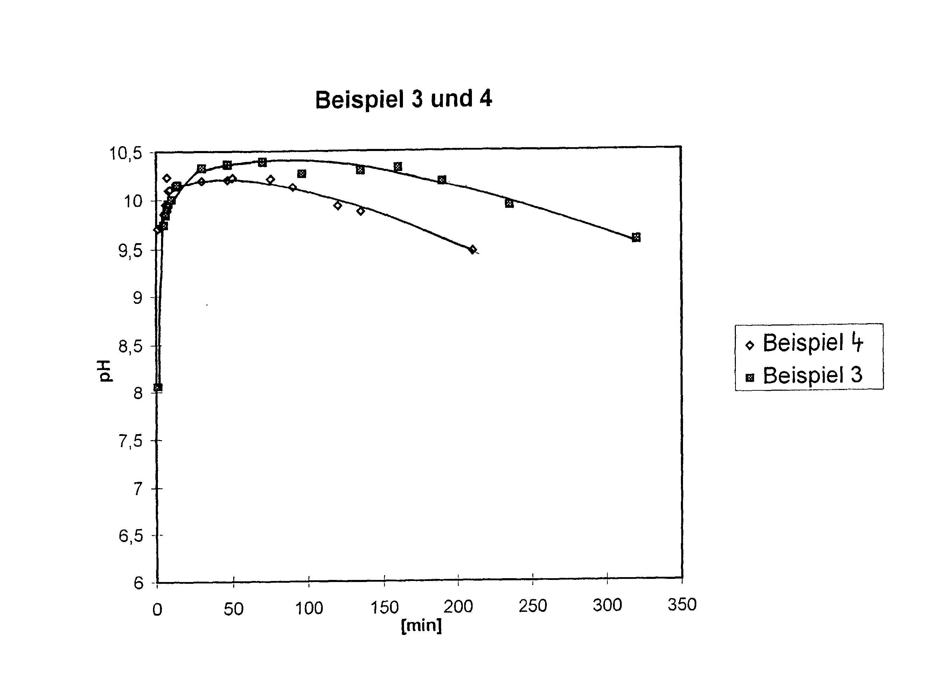

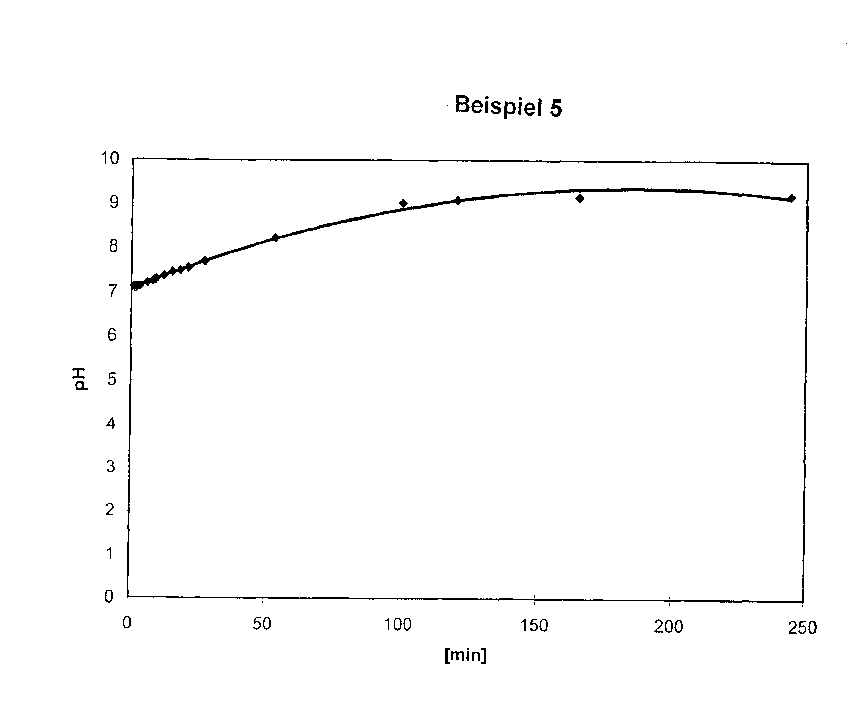

In der Zeichnung zeigen die Fig. 1 bis 4 Diagramme, in denen der pH-Wert der erfindungsgemäßen Mischungen gemäß den Beispielen 1 bis 5 in einem wäßrigen Puffer als Funktion der Zeit aufgezeichnet ist.

Im Rahmen der Ausführungsbeispiele wird der pH-Wert der erfindungsgemäßen Mischungen in einer gepufferten wäßrigen Lösung als Funktion der Zeit gemessen. Diese Messung ist von Bedeutung zur Beurteilung der Wirksamkeit der Förderung der Kollagenneubildung. DE 42 40 713 C1 zufolge führt das Aufbringen einer wäßrigen Calciumhydroxidlösung oder -suspension auf ein Knochentrauma durch den auftretenden sofortigen pH-Sprung zu einer Nekrose des Gewebes. Eine Kollagensynthese ist nicht nachweisbar. Die Kollagenneubildung wird jedoch gefördert, wenn Ca(OH)₂ langsam freigesetzt und ein pH-Wert von etwa 11, bevorzugt 10,5, nicht überschritten wird.

### Herstellung der Pufferlösung.

68 mg Imidazol werden mit destilliertem Wasser verdünnt und mit 0,1 n HCl auf einen pH-Wert von 7 eingestellt. Anschließend wird mit destilliertem Wasser auf 1.000 ml aufgefüllt.

### Beispiel 1

35 g Vaseline (DAB 10), 45 g Rizinusöl und 20 g Ca(OH)₂ werden intensiv gemischt. 6 g dieser Mischung werden unter 30 ml Puffer gelagert und der pH-Wert gemessen.

| Zeit [min] | pH | Zeit [min] | pH |
|---|---|---|---|
| 1 | 7,38 | 6 | 10,5 |
| 2 | 9,3 | 7 | 10,55 |
| 3 | 10,1 | 10 | 10,63 |
| 4 | 10,3 | 15 | 10,71 |
| 5 | 10,42 | 27 | 10,73 |

### Beispiel 2

35 g Vaseline (DAB 10), 45 g Sonnenblumenöl und 20 g Ca(OH)₂ werden dispergiert. 6 g dieser Mischung werden unter 30 ml Puffer gelagert und der pH-Wert gemessen.

| Zeit [min] | pH | Zeit [min] | pH |
|---|---|---|---|
| 1 | 7,5 | 27 | 9,58 |
| 2 | 7,99 | 58 | 9,64 |
| 3 | 8,18 | 90 | 9,58 |
| 4 | 8,41 | 135 | 9,63 |
| 5 | 8,66 | | |

### Beispiel 3

20 g Vaseline (DAB 10), 20 g Jojobaöl und 60 g Ca(OH)₂ werden dispergiert. 6 g dieser Mischung werden unter 30 ml Puffer gelagert und der pH-Wert gemessen.

### Beispiel 4

35 g Vaseline (DAB 10), 45 g Jojobaöl und 20 g Ca(OH)₂ werden dispergiert. 6 g dieser Mischung werden unter 30 ml Puffer gelagert und der pH-Wert gemessen.

| Zeit [min] | Beispiel 4 | Beispiel 3 | Zeit [min] | Beispiel 4 | Beispiel 3 |
|---|---|---|---|---|---|
| 1 | 9,7 | 8,1 | 47 | 10,2 | 10,4 |
| 5 | 9,9 | 9,7 | 90 | 10,1 | |
| 6 | 10,0 | 9,8 | 96 | | 10,3 |
| 7 | 10,2 | 9,9 | 135 | 9,9 | 10,3 |
| 8 | 10,1 | 10,0 | 210 | 9,5 | |
| 30 | 10,2 | 10,3 | 235 | | 9,9 |

### Beispiel 5

17 g Vaseline (DAB 10), 17 g Sonnenblumenöl, 66 g Ca(OH)₂ und 0,1 g 2,6-Di-tert.-butyl-4-methylphenol werden dispergiert. 6 g dieser Mischung werden unter 30 ml Puffer gelagert und der pH-Wert gemessen.

| Zeit [min] | pH | Zeit [min] | pH |
|---|---|---|---|
| 1 | 7,1 | 18 | 7,5 |
| 2 | 7,1 | 21 | 7,6 |
| 3 | 7,1 | 27 | 7,7 |
| 6 | 7,2 | 53 | 8,2 |
| 8 | 7,3 | 100 | 9,0 |
| 9 | 7,3 | 120 | 9,1 |
| 12 | 7,3 | 165 | 9,2 |
| 15 | 7,5 | 244 | 9,2 |

### Lagerstabilität

Die Pasten gemäß den Beispielen 3 und 4 wurden bei 40°C im Wärmeschrank gelagert. Nach einem halben Jahr war keine Konsistenzänderung zu beobachten. Dies läßt auf eine Lagerstabilität von mehr als einem Jahr bei Raumtemperatur schließen.

## Patentansprüche

1. Rinderklauenölfreie Mischung, die enthält
a) Paraffine nach DAB und/oder synthetische Wachse ausgewählt aus der Gruppe bestehend aus Montanwachsen, Wachsen aus Erdöl, Fischer-Tropsch-Wachsen, Polyolefinwachsen, Vaseline, Wachsalkoholen, und Oxidaten der vorgenannten Stoffe;
b) wenigstens ein Metallhydroxid,
zur Verwendung als Wundverband.

2. Mischung nach Anspruch 1, **dadurch gekennzeichnet, daß** sie Vaseline enthält.

3. Mischung nach Anspruch 1 oder 2, **dadurch gekennzeichnet, daß** sie zusätzlich pflanzliche Öle, Fette und/oder Wachse enthält.

4. Mischung nach Anspruch 3, **dadurch gekennzeichnet, daß** sie Jojobaöl, Rizinusöl und/oder Sonnenblumenöl enthält.

5. Mischung nach einem der Ansprüche 1 bis 4, **dadurch gekennzeichnet, daß** sie Alkali- und/oder Erdalkalihydroxide enthält.

6. Mischung nach Anspruch 5, **dadurch gekennzeichnet, daß** sie Calciumhydroxid enthält.

7. Mischung nach Anspruch 6, **dadurch gekennzeichnet, daß** der Calciumhydroxidanteil 10 - 75 Gew.-% beträgt.

8. Mischung nach einem der Ansprüche 3 bis 7, **dadurch gekennzeichnet, daß** der Anteil pflanzlicher Öle, Fette und/oder Wachse 10 - 60 Gew.-% beträgt.

9. Mischung nach einem der Ansprüche 1 bis 8, **dadurch gekennzeichnet, daß** der Anteil synthetischer Wachse und/oder deren Oxidate 10 - 50 Gew.-% beträgt.

10. Mischung nach einem der Ansprüche 1 bis 9, **dadurch gekennzeichnet, daß** sie zusätzlich Füllstoffe enthält.

11. Verwendung einer Mischung nach einem der Ansprüche 1 bis 10 zur Herstellung eines Wurzelkanalfüllmaterials.

## Claims

1. A mixture which comprises
a) paraffins complying with DAB and/or synthetic waxes selected from the group consisting of montan waxes, petroleum waxes, Fischer-Tropsch waxes, polyolefin waxes, petrolatum, wax alcohols, and oxidates of the aforementioned substances;
b) at least one metal hydroxide,
the mixture containing no neatsfoot oil, for use as wound dressing.

2. A mixture as claimed in claim 1, which comprises petrolatum.

3. A mixture as claimed in claim 1 or 2, which additionally comprises vegetable oils, fats and/or waxes.

4. A mixture as claimed in claim 3, which comprises jojoba oil, castor oil and/or sunflower oil.

5. A mixture as claimed in any of claims 1 to 4, which comprises alkali metal and/or alkaline earth metal hydroxides.

6. A mixture as claimed in claim 5, which comprises calcium hydroxide.

7. A mixture as claimed in claim 6, wherein the calcium hydroxide content is 10-75% by weight.

8. A mixture as claimed in any of claim 3 to 7, wherein the content of vegetable oils, fats and/or waxes is 10-60% by weight.

9. A mixture as claimed in any of claims 1 to 8, wherein the content of synthetic waxes and/or their oxidates is 10-50% by weight.

10. A mixture as claimed in any of claims 1 to 9, which additionally comprises fillers.

11. The use of a mixture as claimed in any of claim 1 to 10 for preparing a root canal filling material.

## Revendications

1. Mélange dépourvu d'huile de pieds de boeuf, contenant
a) de la paraffine selon le DAB et/ou de la cire synthétique choisie dans le groupe comprenant les cires de lignite, cires de pétrole, cires de Fischer-Tropsch, cires de polyoléfines, vaselines, alcools de cire et oxydats des substances précédentes ;
b) au moins un hydroxyde métallique,
pour l'utilisation comme pansement d'une plaie.

2. Mélange selon la revendication 1, **caractérisé en ce qu'**il contient de la vaseline.

3. Mélange selon la revendication 1 ou 2, **caractérisé en ce qu'**il contient en outre des huiles végétales, des graisses et/ou des cires.

4. Mélange selon la revendication 3, **caractérisé en ce qu'**il contient de l'huile de jojoba, de l'huile de ricin et/ou de l'huile de tournesol.

5. Mélange selon l'une quelconque des revendications 1 à 4, **caractérisé en ce qu'**il contient des hydroxydes alcalins et/ou alcalino-terreux.

6. Mélange selon la revendication 5, **caractérisé en ce qu'**il contient de l'hydroxyde de calcium.

7. Mélange selon la revendication 6, **caractérisé en ce qu'**il comprend 10 à 75 % en poids de proportion d'hydroxyde de calcium.

8. Mélange selon l'une quelconque des revendications 3 à 7, **caractérisé en ce que** la proportion d'huiles végétales, de graisses et/ou de cires est de 10 à 60 % en poids.

9. Mélange selon l'une quelconque des reveridications 1 à 8, **caractérisé en ce que** la proportion de cires synthétiques et/ou de leurs oxydats est de 10 à 50 % en poids.

10. Mélange selon l'une quelconque des revendications 1 à 9, **caractérisé en ce qu'**il contient en outre des substances de remplissage.

11. Utilisation d'un mélange selon l'une quelconque des revendications 1 à 10, pour produire un matériau de remplissage des canaux radiculaires.
